# EUROPEAN PATENT APPLICATION

(11) **EP 4 220 169 A1**
(43) Date of publication of application: **02.08.2023**
(21) Application number: 22382079.6
(22) Date of filing: 31.01.2022
(51) Int. Cl.: G01N 33/574

(54) **IN VITRO METHOD FOR THE DIAGNOSIS AND/OR PROGNOSIS OF PITUITARY NEUROENDOCRINE TUMOURS**

(71) Applicant: Universidad Autónoma de Madrid, 28049 Madrid (ES); Fundación de Investigación Biomédica del Hospital Universitario de la Princesa, 28006 Madrid (ES)
(72) Inventor: MARTÍNEZ HERNÁNDEZ, Rebeca, 28049 Madrid (ES); MARAZUELA AZPIROZ, Mónica, 28049 Madrid (ES); SERRANO SOMAVILLA, Ana, 28006 Madrid (ES); SANZ GARCIA, Ancor, 28006 Madrid (ES); SAMPEDRO NUÑEZ, Miguel Antonio, 28006 Madrid (ES)
(74) Representative: Hoffmann Eitle

(57) **Abstract**

The present invention refers to an *in vitro* method for the diagnosis and/or prognosis of pituitary neuroendocrine tumours (PitNETs) in a subject. The invention is also focused on an *in vitro* method for monitoring the aggressiveness of PitNETs and for the differential diagnosis of non-functioning PitNET versus functioning PitNET.

## Description

### FIELD OF THE INVENTION

The present invention refers to the medical field. Particularly, it refers to an *in vitro* method for the diagnosis and/or prognosis of pituitary neuroendocrine tumours (PitNETs) in a subject. The invention is also focused on an *in vitro* method for monitoring the aggressiveness of PitNETs and for the differential diagnosis of non-functioning PitNET versus functioning PitNET.

### STATE OF THE ART

Pituitary neuroendocrine tumours (PitNETs)are monoclonal tumours arising from the neuroendocrine epithelial cells of the adenohypophysis that represent more than 10% of all intracranial neoplasms. PitNETss are classified according to their function into either functional or non-functional adenomas (NF-PitNETs). Functional adenomas can result in distinct hypersecretory syndromes that depend on the cell type of origin. Corticotropin-secreting adenomas result in Cushing's disease, growth hormone-secreting adenomas in acromegaly, prolactin-secreting adenomas in hyperprolactinemia, and thyrotropin-secreting adenomas in hyperthyroidism. In contrast, the diagnosis of NF-PitNETss is clinically challenging, since they do not produce a hormonal hypersecretion syndrome, and are usually diagnosed either incidentally or in relation to local compressive symptoms such as headache and visual field defects. Gonadotroph adenomas can cause hypogonadism but they usually appear as non-functioning presenting with an incidental sellar mass. PitNETs can sometimes exhibit a clinically aggressive behaviour with invasion of the surrounding tissues and/or recurrence after surgery. Except for prolactinomas, the treatment of choice for PitNETs is surgical removal of the tumour. Medical treatment is reserved for cases of incomplete resection, recurrence, or surgical contraindication. In these situations, available medical therapies include dopamine receptor type 2 (DRD2) agonists (DAs) (mostly in prolactinomas and occasionally in acromegaly), adrenal enzyme inhibitors (in Cushing's disease) and somatostatin analogues (SSA) (in acromegaly, thyrotropinomas, and Cushing's disease). Several studies have evaluated potential predictors of biochemical and radiological response to SSA including age, sex, cortisol and growth hormone (GH) levels, the size of the adenoma, and presence or absence of tumour invasion. Although the pathogenic mechanisms of pituitary tumorigenesis remain unknown, different molecular mechanisms including genetic and epigenetic changes have been described to contribute to pituitary neoplasia initiation. The involved mechanisms include cell cycle dysregulation, activation of oncoproteins, alterations in growth factor signalling, changes in the intra-pituitary microenvironment, and germline or somatic mutations.

Primary cilia (PC) are antenna-like organelles that project from the cell membrane to the extracellular environment and consist of a microtubule-based core structure that elongates from the basal body. PC are considered as crucial sensors providing the ability to fine-tune cell signalling and thus key cellular processes. Malfunctioning cilia can therefore be the cause of a diverse set of diseases called ciliopathies. Recently, an emerging role of the PC in the regulation of cancer development has been described. The PC function as a control centre for different signalling pathways associated with tumorigenesis, including the Hedgehog (Hh), the Wnt, and the PDGF signalling pathways. In addition, a close relationship between PC and the cell cycle has been reported.

Cells from the different endocrine organs possess PC that can localise with hormone receptors. Growing evidence has highlighted the association of these organelles with endocrine-related cancers. In this regard, PC loss appears to be a consistent feature of breast, ovarian and prostate cancer albeit in a limited number of relatively small studies. Regarding other endocrine-related tumours, although PC are present on multiple cell types including pituitary, pancreas, adrenal, thyroid and testis cells, no data or experimental evidence directly link PC and neoplasms of these endocrine organs.

Finally, kindly note that there is an unmet medical need of finding new strategies for the diagnosis and/or prognosis of PitNETs, and for monitoring the aggressiveness of these tumours. The present invention is thus focused on solving this problem, an innovative method for the diagnosis and/or prognosis of PitNETs, and for monitoring the aggressiveness of PitNETs, is herein provided.

### DESCRIPTION OF THE INVENTION

### Brief description of the invention

As cited above, the present invention refers to an *in vitro* method for the diagnosis and/or prognosis of PitNETs in a subject. The invention is also focused on an *in vitro* method for monitoring the aggressiveness of PitNETs and for the differential diagnosis of non-functioning PitNET versus functioning PitNET.

Particularly, according to the inventors of the present invention, the study of cilia could serve as a diagnostic tool providing new insights into the mechanisms of tumorigenesis and aggressiveness of PitNETs. So, in the context of the present invention, a total of 86 patients with PitNETs (28 functioning and 58 non-functioning [NF-PitNETs]) and 12 controls were evaluated by immunofluorescence and immunohistochemistry in tissue microarrays (TMA) and by western blot in PitNET protein extracts. The frequency of ciliated cells was estimated using a cilia index score based on the length of cilia and the percentage of ciliated area in the tissue. The distribution of PC and its correlation with several clinical parameters and aggressiveness was analysed. In addition, the expression levels of Gli1 (a component of the Hedgehog pathways) and the stem cell marker Sox2 and their correlation with the cilia index were evaluated in PitNETs. According to the results provided in the present invention (see **Example 2** below) PC were present only in scattered cells of control pituitary tissues, whereas PitNET cells showed robust staining for ciliary markers. Interestingly, the number and /or length of ciliated cells was significantly higher in non-functioning PAs, including null cell adenomas and gonadrotropinomas, when compared to functional tumours. Remarkably, PC were significantly increased in non-functioning-PitNETs and were associated with tumour invasion, aggressiveness and recurrence in PitNETs. Moreover, changes in ciliation positively correlated with the expression of the transcription factor Gli1 and the stem cell marker Sox2. So, in conclusion, the present invention shows that PC represent an important contributor to aggressiveness in PitNETs and may serve as a novel diagnostic marker for predicting tumour behaviour.

So, the first embodiment of the present invention refers to an *in vitro* method for the diagnosis and/or prognosis of PitNETs in a subject, which comprises assessing the presence of PC in cells from a biological sample obtained from the subject, wherein the identification of a frequency of ciliated cells higher than a pre-established frequency threshold value measured in control subjects, is an indication that the subject is suffering from PitNETs and/or that the subject has a poorer prognosis.

The second embodiment of the present invention refers to an *in vitro* method for monitoring the aggressiveness of PitNETs which comprises assessing the presence of PC in cells from a biological sample obtained from the subject, wherein the identification of a frequency of ciliated cells higher than a pre-established frequency threshold value measured in control subjects, is an indication that the disease is more aggressive.

The third embodiment of the present invention refers to an *in vitro* method for the differential diagnosis of non-functioning PitNET versus functioning PitNET which comprises assessing the presence of PC in cells from a biological sample obtained from the patient, wherein the identification of a frequency of ciliated cells higher than a pre-established frequency threshold value measured in control subjects, is an indication that the subject is probably suffering from a non-functioning PitNETs.

In a preferred embodiment of the present invention, the PitNET is a non-functioning PitNET.

In a preferred embodiment of the present invention, the identification of ciliated cells is carried out by immunohistochemical or immunofluorescence methods or by electron microscopy.

In a preferred embodiment of the present invention, the identification of ciliated cells is carried out by using an antibody against ADP ribosylation factor like GTPase 13B.

In a preferred embodiment of the present invention, the biological tissue sample is a pituitary sample. In other word, presence of PC in cells would be assessed after obtaining a biopsy of the pituitary tumor.

The fourth embodiment of the present invention refers to the *in vitro* use of PC in cells from a biological sample obtained from the patient for the diagnosis and/or prognosis of PitNETs, for monitoring the aggressiveness of PitNETs, or for the differential diagnosis of non-functioning pituitary adenoma versus functioning pituitary adenoma.

The fifth embodiment of the present invention refers to the *in vitro* use of a kit consisting of tools for the identification of primary cilia in cells from a biological sample obtained from the patient, for the diagnosis and/or prognosis of PitNETs, for monitoring the aggressiveness of PitNETs, or for the differential diagnosis of non-functioning pituitary adenoma versus functioning pituitary adenoma.

Alternatively, the present invention also refers to a method for assessing the presence of ciliated cells in a biological sample obtained from a subject at risk of developing PitNETs, or a patient suffering from PitNETs, wherein the identification ciliated cells is carried out by immunohistochemical or immunofluorescence methods, by electron microscopy or by using an antibody against ADP ribosylation factor like GTPase 13B.

The present invention also refers to a method for analysing a biological sample prior to diagnosis or prognosis of PitNETs, comprising analysing a test sample for the presence of ciliated cells, wherein the identification of a frequency of ciliated cells higher than a pre-established frequency threshold value measured in control subjects is indicative of the biological sample containing a population of malignant cells.

The present invention also refers to a method for treating a patient suffering from PitNETs, wherein the patient has been diagnosed following the above method described in the first embodiment of the invention.

In a preferred embodiment, the present invention is a computer-implemented invention, wherein a processing unit (hardware) and a software are configured to:
- Receive the frequency of ciliated cells detected in a biological sample,
- Process the frequency of ciliated cells received for finding substantial variations or deviations, and
- Provide an output through a terminal display of the variation or deviation of the frequency of ciliated cells, wherein the variation or deviation of the frequency of ciliated cells indicates that the subject may be suffering from PitNETs or has a poor prognosis.

Moreover, the present invention also comprises a computer program or a computer-readable media containing means for carrying out a method defined above.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of' means including, and it is limited to, whatever follows the phrase "consisting of'. Thus, the phrase "consisting of' indicates that the listed elements are required or mandatory, and that no other elements may be present.
- The term pituitary neuroendocrine tumours (PitNETs) embraces the term Pituitary Adenomas (PAs) which are tumours arising from the neuroendocrine epithelial cells of the adenohypophysis. The following refences deals with the nomenclature evolution of this term and with its definition [Sylvia L Asa, et al., 2020. Pituitary neuroendocrine tumors (PitNETs): nomenclature evolution, not clinical revolution. Pituitary. 2020 Jun;23(3):322-325. doi: 10.1007/s11102-019-01015-0. PMID: 31834538 DOI: 10.1007/s11102-019-01015-0] [Abdellah Tebani et al., 2021. Annotation of pituitary neuroendocrine tumors with genome-wide expression analysis. Acta Neuropathologica Communications volume 9, Article number: 181 (2021*)*] [New WHO classification of pituitary adenomas (4th edition): assessment of pituitary transcription factors and the prognostic histological factors. Hiroshi Nishioka, Naoko Inoshita. Brain Tumor Pathol. 2018 Apr;35(2):57-61. doi: 10.1007/s10014-017-0307-7]*.*
- The term "pre-established frequency threshold value measured in control subjects" typically refers to the frequency measured in healthy subjects (subjects who are not suffering from PitNETs) when a method for the diagnosis of the disease is envisaged or measured in patients when the intention is performing a method for the prognosis of the disease. The patient is likely to suffer from PitNETs with a given sensitivity and specificity if the levels of the frequency of ciliated cells are above said "pre-established threshold level". A "threshold value" or "cut-off value" can be determined experimentally, empirically, or theoretically. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Thus, in one embodiment of the present invention, the "threshold value" is derived from the frequency of ciliated cells determined in a control sample derived from one or more subjects who are substantially healthy. In one embodiment of the present invention, the "threshold value" may also be derived from the frequency of ciliated cells determined in a control sample derived from one or more subjects who suffers from PitNETs. Furthermore, retrospective measurement of the frequency of ciliated cells in properly banked historical subject samples may be used in establishing these "threshold values". Typically, the optimal sensitivity and specificity (and so the "threshold value") can be determined using a Receiver Operating Characteristic (ROC) curve.

### Description of the figures

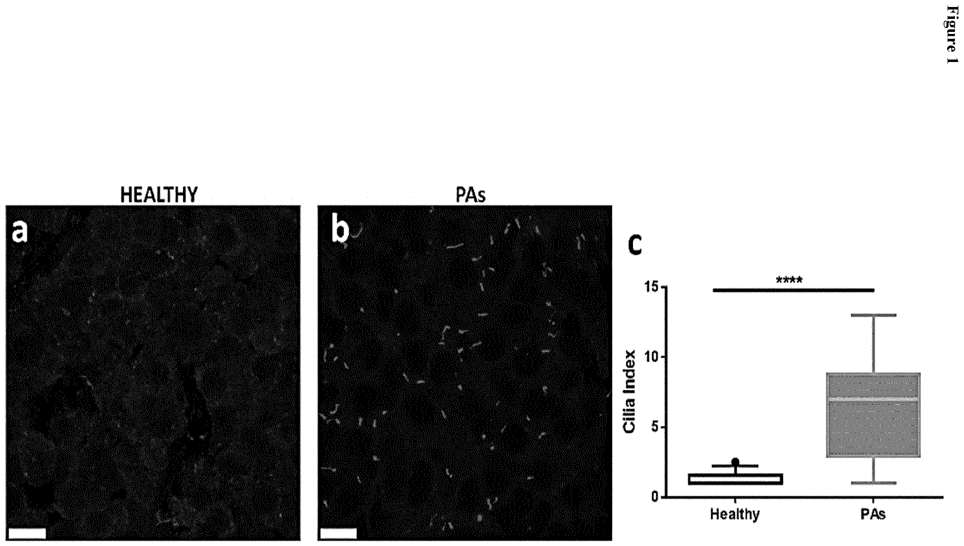

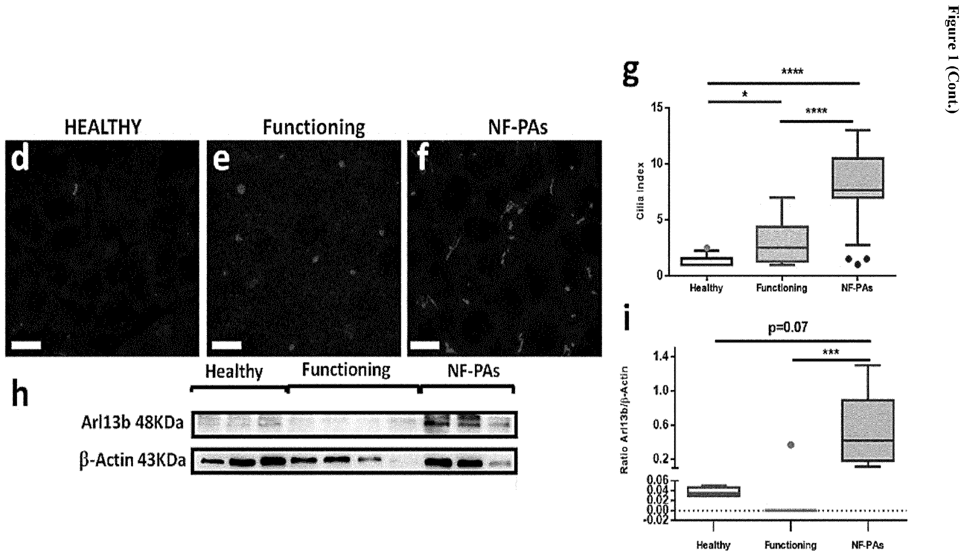

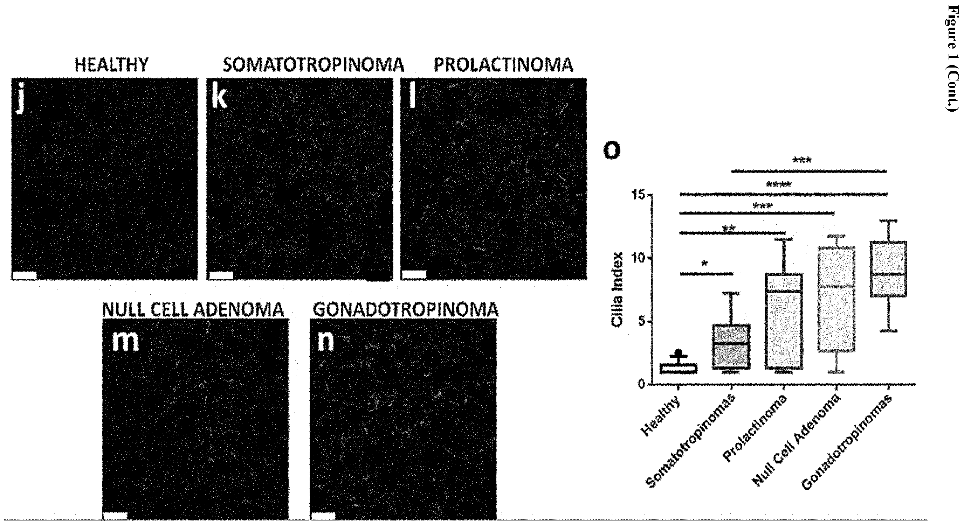

**Figure 1****. Cilia patterning in pituitary tissue and in PitNETs.** Representative images captured by confocal microscopy characterising the primary cilia of controls **(a)** and PitNETs **(b)** stained with Arl13b (in red) and nuclei counterstained with DAPI (blue). Scale bar=10 µm. Box and whisker plot displaying the quantification of primary cilia by the cilia index score, as reported in Material and Methods section, in a total of 172 TMA samples **(c)**. PC details of pituitary glands from the different groups analysed: functioning- vs non-functioning-PitNETs **(d-f)**. Scale bar= 7.5 µm. Box and whisker plot displaying the quantification of primary cilia in the three groups analysed **(g)**. Western blot and quantification of AR113b in protein extracts from healthy, functioning and NF-PitNET pituitary tissues **(h)** and **(i)**. Representative immunofluorescence staining of Arl13b (in red) of control pituitary tissue, somatotropinoma, prolactinoma, null cell adenoma and gonadotropinoma pituitary tumours. Scale bar=10 µm **(j-n)**. Box plots representing cilia index in the different types of PitNETs **(o)**. Data correspond to the median (lines within boxes), interquartile range (bounds of boxes), and upper and lower range (whiskers). Differences were analysed by one-way ANOVA test. -ns, not significant; ^{∗}p <0.05; ^{∗∗}p<0.01; ^{∗∗∗}p<0.005; ^{∗∗∗∗}p<0.001.

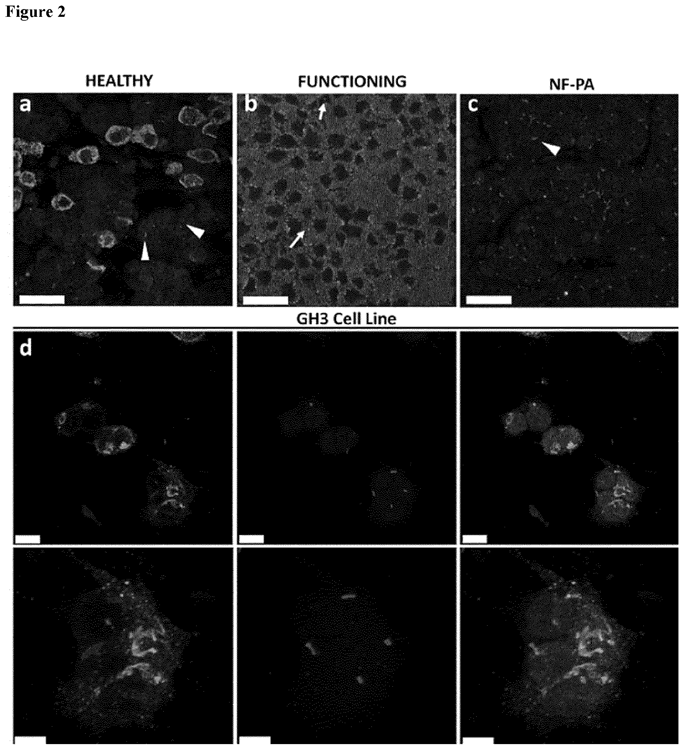

**Figure 2****. Triple immunofluorescence staining for growth hormone cilia and nuclei in healthy pituitary tissue and pituitary adenomas.** Healthy pituitary tissue **(a)**, functioning PitNETs **(b)**, NF-PitNETs **(c)** and GH3 cell line **(d)** were stained for GH (green), cilia (Arl13b, red) and nuclei (DAPI, blue). Representative immunofluorescence images are shown. Arrowheads points to "normal-like" cilia, arrows indicate "dot-like" cilia. In healthy tissues cilia appeared mainly in GH-negative cells, whereas in PitNETs they appear both in GH-positive and negative cells. Scale Bar= 25 µm. In the GH3 cell line, both GH-positive and GH-negative cells presented PC. Scale Bar=10 µm and 5 µm.

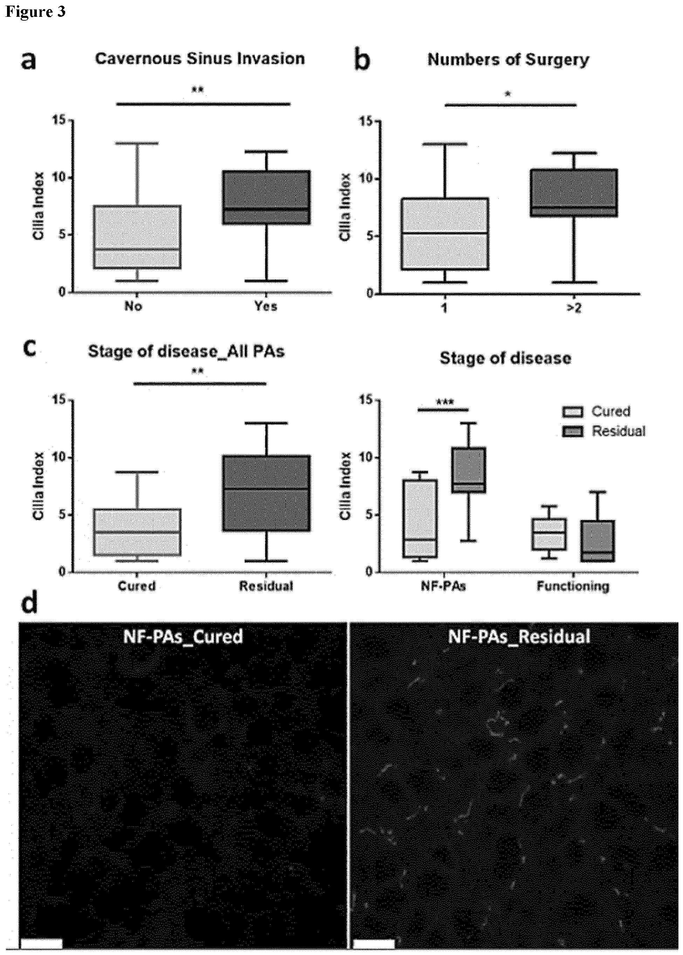

**Figure 3****. Cilia in aggressive adenomas.** Tissues from PitNETs were stained for Arl13b and cilia were analysed by immunofluorescence. Cilia index was quantified and analysed according to cavernous sinus invasion (No invasion vs Invasion) **(a),** number of surgeries (1 vs >2) **(b)** and stage of disease (cured vs residual) **(c)** in PitNETs. Representative images of cilia (red) in cured and residual NF-PitNETs are shown **(d),** scale bar=10 µm. Data correspond to the median (lines within boxes), interquartile range (bounds of boxes), and upper and lower range (whiskers). Differences were analysed by Mann-Whitney U test. ns, not significant; ^{∗}p <0.05; ^{∗∗}p<0.01; ^{∗∗∗}p<0.005; ^{∗∗∗∗}p<0.001.

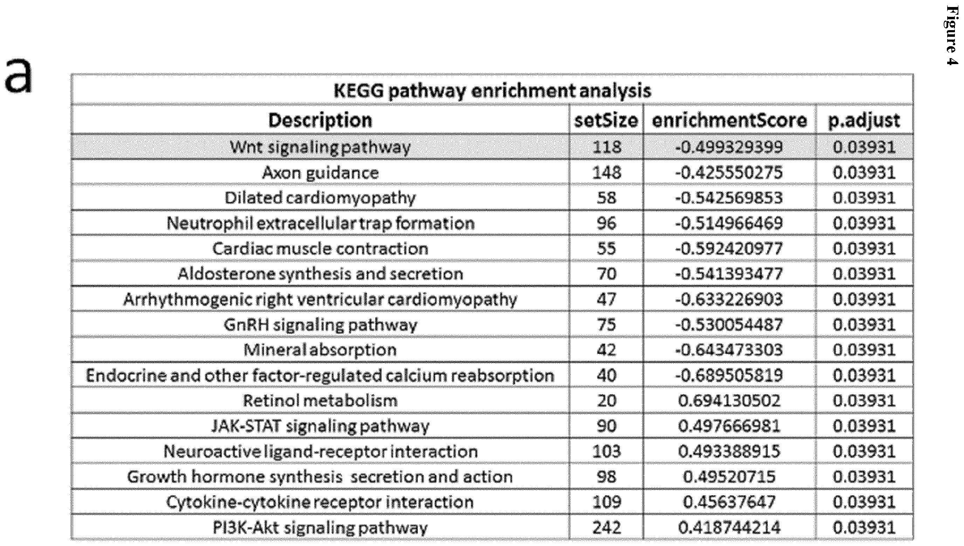

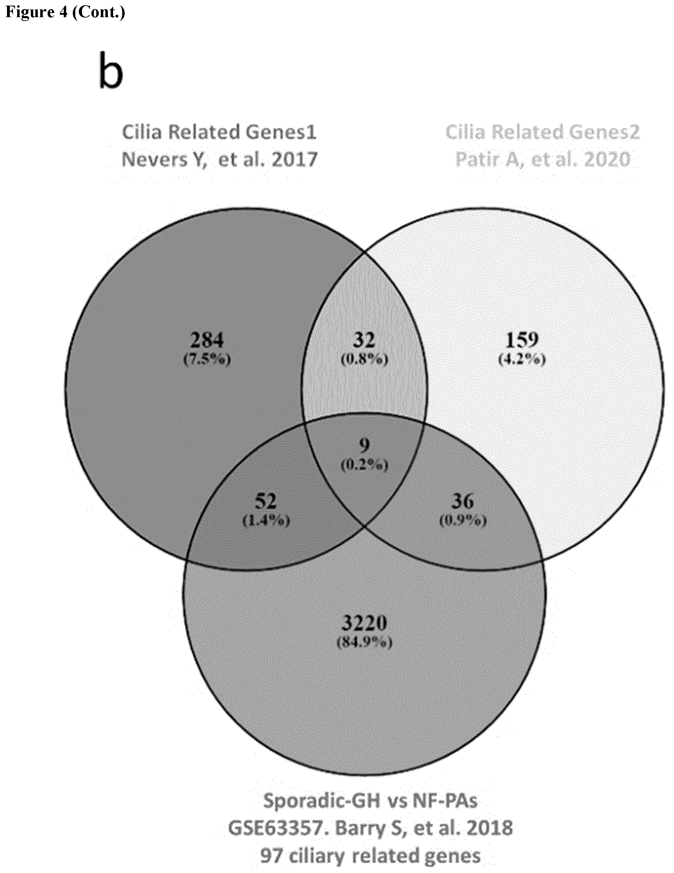

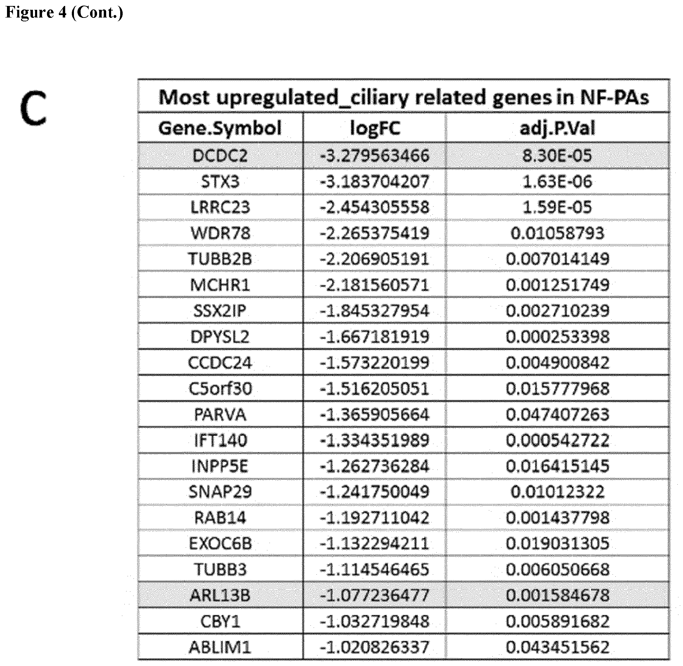

**Figure 4****. Cilia-associated gene profiling. a)** Table representing the KEGG pathway analysis of the genes differentially expressed between somatotropinomas and NF-PitNETs. The most represented network is the Wnt signalling pathway. **b)** Venn diagram representing the genetic set relations between two ciliary genetic dataset profiles and the differential transcriptomic profile between Sporadic GH-secreting adenomas and NF-PAs from the dataset downloaded from the Gene Expression Omnibus (GEO accession number GSE63357). **c)** Table list of the most upregulated cilia-related genes in NF-PitNETs in comparison with Sporadic GH-secreting adenomas. Dyslexia Candidate Gene DCDC2 is the most upregulated gene.

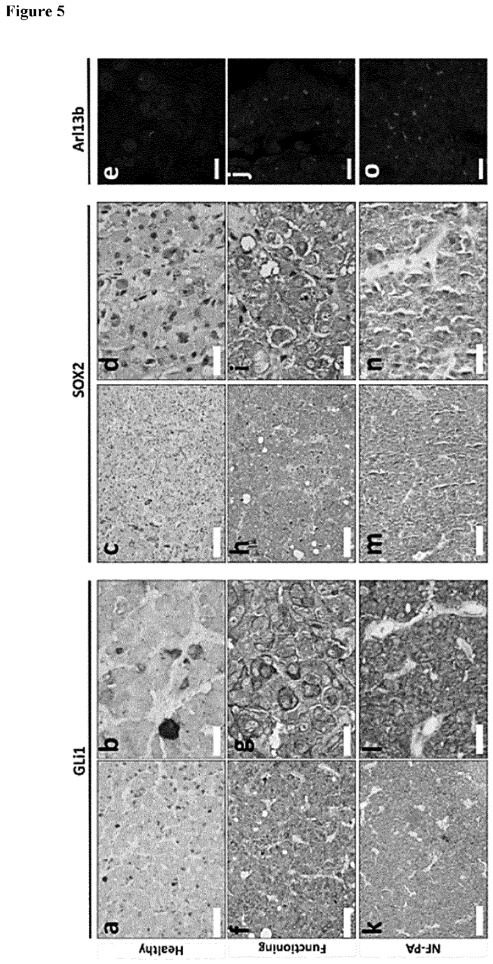

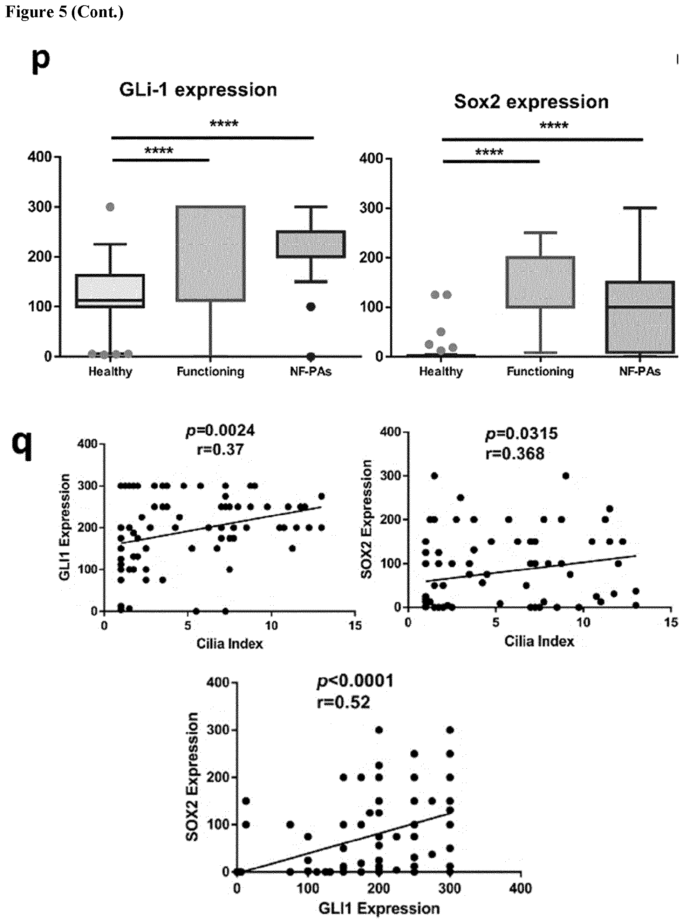

**Figure 5****. Correlation of Glil and Sox2 expression with Cilia Index.** Representative images of Glil and Sox2 immunohistochemistry in healthy **(a-d)** and PitNET tissues (**f-I** and **k-n**). Immunofluorescence images of the same tissue showing staining for PC (Arl13b, in red) and nuclei (DAPI, in blue) (e, j and o). Scale bars: a, c, f, h, k and m= 200µm; b, d, g, i, 1 and n= 50µm; e, j and o= 10µm. Quantitative analysis of Glil and Sox2 expression in TMAs **(p).** PitNETs had a significant increase in expression of both transcription factors compared to healthy tissue (p<0.0001, p). Positive correlation analysis **(q)** between Glil and cilia index (p=0.024), Sox2 and Cilia Index (p=0.0315) and Glil and Sox2 expression (p<0.0001). Differences were analysed by one-way ANOVA test and correlations between the different markers were performed by Spearman's rho analyses. -ns, not significant; ^{∗}p <0.05; ^{∗∗}p<0.01; ^{∗∗∗}p<0.005; ^{∗∗∗∗}p<0.001.

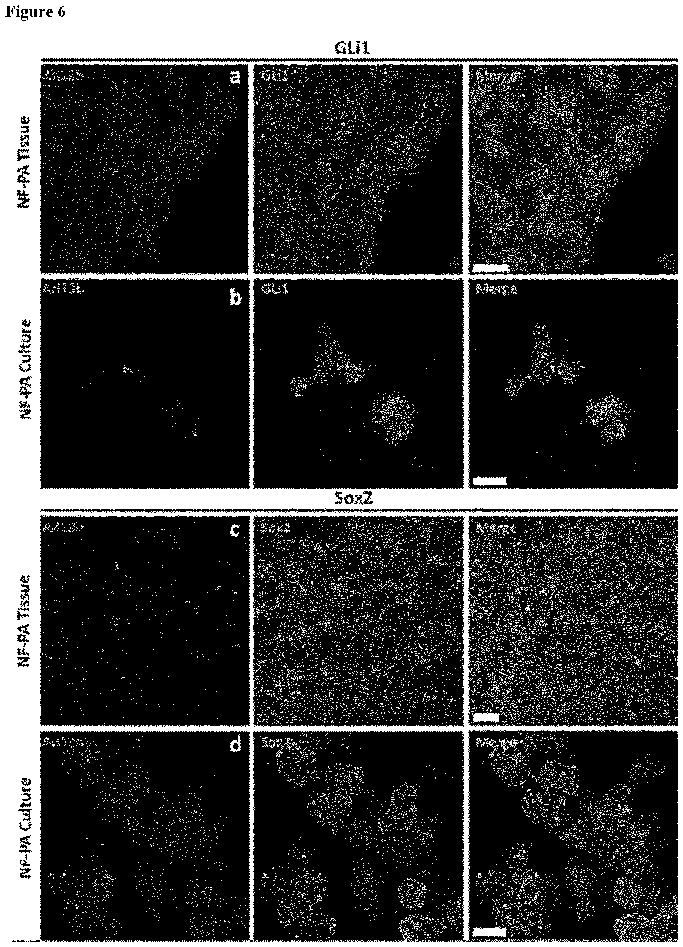

**Figure 6****. Glil and Sox2 localisation in tissue and primary culture of NFPA.** Tissue Gli1 was highly expressed in the cytoplasm, with some cells showing a strong expression at the end of the PC **(a).** Primary culture cells derived from NF-PitNET also showed high Glil expression in ciliated cells **(b).** Sox2 expression was distributed throughout the NFPitNET tissue **(c)** and presented an increase Sox2 expression those cells with PC **(d).**

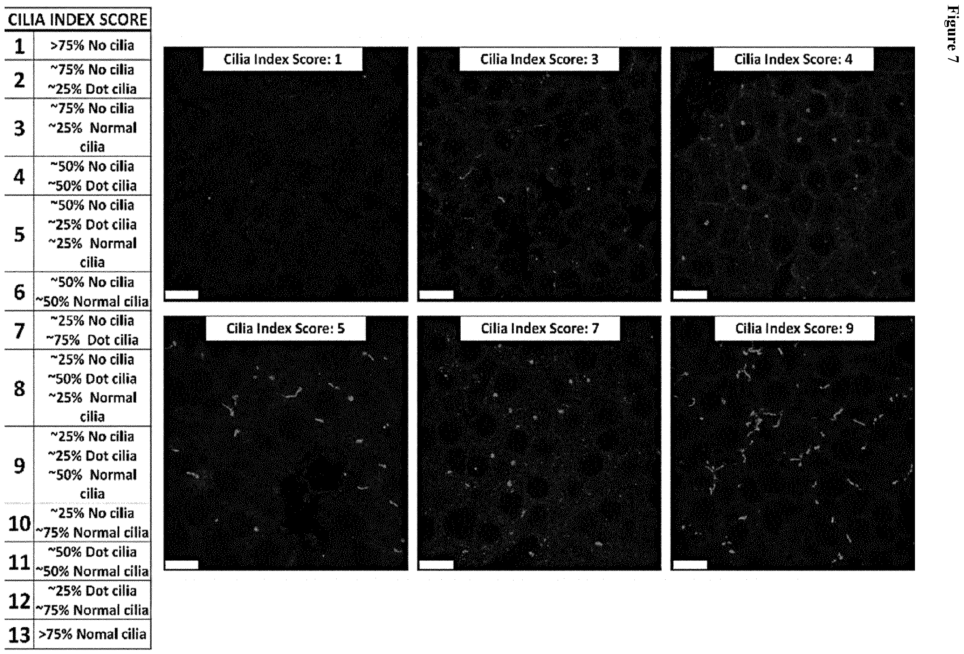

**Figure 7****. Cilia index score.** Cilia index assessed from immunofluorescence staining of cilia in tissue sections as described in materials and methods section according to cilia length (dot-like cilia and normal cilia) and percentage of ciliated area on the tissue. Right, table detailing criteria to define the different cilia index scores. Left, representative images of different cilia index scores.

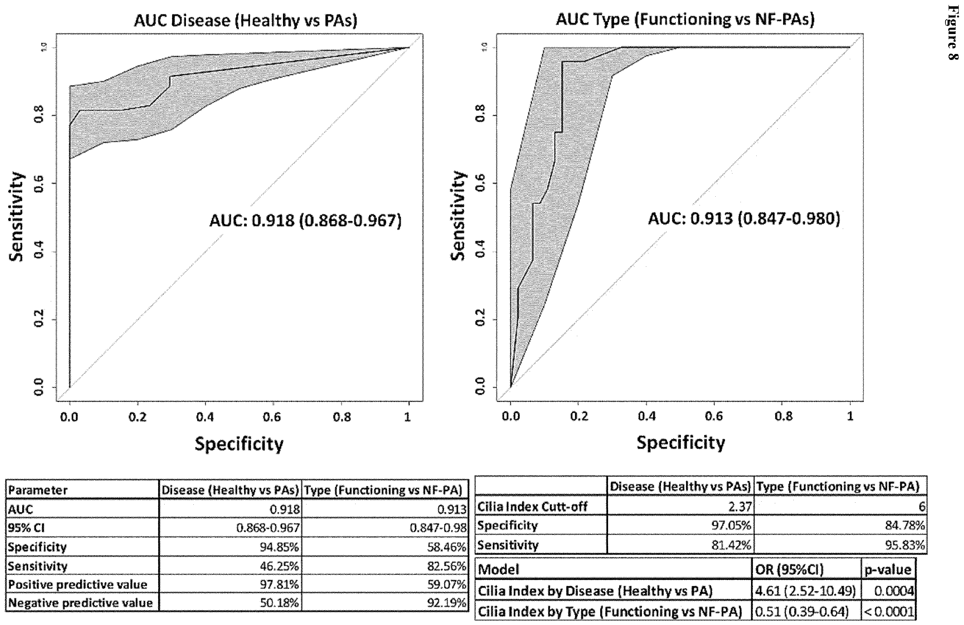

**Figure 8****.** ROC curves showing AUC values for the parameter healthy vs PitNETs and functionating vs NF-PA.

### Detailed description of the invention

The present invention is illustrated by means of the Examples set below without the intention of limiting its scope of protection.

### Example 1. Material and Methods

### Example 1.1. Individuals

A retrospective study was conducted including consecutive patients with pituitary adenomas (somatotroph adenoma and non-functioning pituitary tumours [NF-PitNETs]) with available tumour samples from different centres in Spain between 1996 and 2018. 86 patients with PitNETs were studied (27 somatotroph adenomas and 59 NF-PitNETs) and 12 normal pituitary glands from autopsy specimens. Immunohistochemical evaluation, imaging and clinical phenotype characteristics are summarised in **Table 1.** PitNETs were classified according to clinically functionality (functioning or non-functioning), size, cavernous sinus invasion, sign intensity in T1 and T2 sequences on magnetic resonance, visual field examination, interpreted by expert endocrinologists. All tumours were also reviewed by the same pathologist and were classified according to the immunohistochemical expression of adenohypophyseal hormones (growth hormone [GH], prolactin [PRL], follicle-stimulating [FSH], luteinizing [LH], thyroid stimulating hormone [TSH], and adrenocorticotroph hormone [ACTH]) **(Table 1**). Patients were classified according to aggressiveness / aggressive features regarding the stage of the disease as cured or controlled (no residual disease or controlled after treatment) and patients with residual/relapsed disease (residual disease and/or recurrence after treatment). This project was approved by the Internal Ethics Review Committee of the Hospital de La Princesa and written informed consent was obtained from all participants prior to inclusion, in accordance with the Declaration of Helsinki.

**Table 1**

| | **Total number of samples (n=86) (%)** | |
|---|---|---|
| **Gender n** = **86** | | |
| Male | 37 (43%) | |
| Female | 49 (57%) | |
| **Age, years (at diagnosis) n=86** | | |
| <55 | 43 (50%) | |
| ≥55 | 43 (50%) | |
| **Clinical diagnosis n=86** | | |
| Functioning | 28 (33%) | |
| Non-Functioning | 58 (67%) | |
| **Anatomopathological diagnosis n=79** | | |
| Gonadotropinomas | 26 (33%) | |
| Prolactinomas (including mixed GH and PRL) | 11 (14%) | |
| Null Cell Adenoma | 15 (19%) | |
| Silent ACTH | 2 (3%) | |
| Somatotropinomas | 24 (31%) | |
| **Stage of disease n=84** | | |
| Cured or controlled | 22 (26%) | |
| Residual tumour | 62 (74%) | |
| **Tumour size, cm n=85** | | |
| Microadenoma <1.0 | 6 (7%) | |
| Macroadenoma ≥1.0 | 79 (93%) | |
| **Numbers of surgeries n=86** | | |
| 1 | 64 (74%) | |
| ≥2 | 22 (26%) | |
| **Cavernous sinus invasion n=85** | | |
| Unilateral | 30 (35%) | |
| Bilateral | 15 (18%) | |
| No invasion | 40 (47%) | |
| **T1/T2 Signal Intensity n=65** | **T1** | **T2** |
| Hypointense | 14 (22%) | 13 (20%) |
| Intermediate | 38 (58%) | 17 (26%) |
| Hyperintense | 11 (17%) | 24 (37%) |
| Hetero geneous | 2 (3%) | 7 (11%) |

| | | |
|---|---|---|
| ***Clinicopathological features of the study population*** | | |

### Example 1.2. Tissue samples

A total of 172 formalin-fixed, paraffin-embedded tissues were evaluated by tissue microarray (TMA). Of these, 158 were tumour samples with pathological diagnosis of PitNET and 14 corresponded to healthy pituitary samples. All samples had a duplicate in the same TMA and were taken and managed in accordance with local regulations with the approval of the local Institutional Review Board.

### Example 1.3. Immunofluorescence and Immunohistochemistry

4µm TMA sections from PitNETs were placed in an oven at 65°C for 30 minutes. Sections were then deparaffinised in xylene and rehydrated through graded alcohols. Antigen retrieval was performed in a PTLink instrument using EnVision Flex target retrieval solution high pH (Dako). For immunofluorescence, non-specific binding was blocked using 5% bovine serum albumin and 10% normal goat serum for 30 minutes. Primary antibodies against ADP ribosylation factor like GTPase 13B (Arl13B) (Proteintech) and Growth Hormone (GH, Bio-Techne R&D Systems) were incubated overnight at 4_{°}C. The following day, an Alexa 568 labelled donkey anti-rabbit (Invitrogen) and an Alexa 488 labelled donkey anti-goat (Invitrogen) were used as secondary antibodies, and nuclei were counterstained by 4',6-diamidino-2-phenylindole (DAPI). For cilia quantification, high resolution images were acquired from 5-7µm-thick stacks in a Leica SP5 confocal microscope (Leica Microsystems). For immunohistochemistry, endogenous peroxidase was inhibited with a peroxidase-blocking solution (Dako) for 10 minutes. Afterwards, sections were immunostained with anti-Sox2 or anti-Glil (ThermoFisher Scientific) overnight at 4°C. Next, sections were incubated with the proper horseradish peroxidase-conjugated secondary antibodies: goat anti-mouse (Dako) or rabbit anti-goat (Ref: P0449, Dako). Finally, sections were incubated with 3,3'-diaminobenzidine (DAB; Dako), counterstained with hematoxylin (Sigma-Aldrich), dehydrated in alcohol, cleared with xylene, and mounted.

### Example 1.4. Marker quantification in TMAs

The frequency of cilia in a total of 172 formalin-fixed, paraffin-embedded tissues was estimated manually by analysing Z-stacked images in confocal microscope. For each tissue all the area of the TMA was analysed by two independent observers in a blinded manner, at higher magnification resolution. The frequency of ciliated cells was estimated using a cilia index score (CI). CI was assigned according to the cilia length appearance (categorised as dot-like cilia and normal-like cilia), and the percentage of ciliated area on the TMA tissue (categorised as ~25%, ~50%, ~75% and >75% of ciliated cells). CI was quantified according to a 1 to 13 scale as detailed in **Figure 7**; values were obtained using the mean of 4 values (2 viewers per 2 duplicated samples).

For Glil and Sox2 quantification a staining score was determined as previously described [*Sampedro-Núnẽz M, Serrano-Somavilla A, Adrados M, et al. Analysis of expression of the PD-1*/*PD-L1 immune checkpoint system and its prognostic impact in gastroenteropancreatic neuroendocrine tumors. Sci Rep 2018;8:17812*] using the approximate percentage of positive cells and staining intensity. All the tissue area from each TMA slide underwent observer-blind examination. The proportion of positively stained cells were scored as follows: <5% stained cells; 5%-25% stained cells; 25%-50% stained cells; 50%-75% stained cells and >75% stained cells. Overall stain intensity was graded as follows: 0, for negative staining; 1, for light staining; 2, for moderate staining, and 3, for intense staining. The total staining score for each field was obtained by multiplying the percentage of stained cells by the staining intensity score.

### Example 1.5. Cell Cultures and reagents

GH3 cells (Merck) were maintained in Ham's F-10 Nutrient Mixture (F-10) supplemented with 2mM glutamine, 15% horse serum (Thermofisher Scientific), 2.5% foetal bovine serum (FBS, HyClone) and 1% penicillin/streptomycin.

NFPitNET cells were prepared from two pituitary gland surgical fragments. In brief, pituitary tissue was minced and digested with collagenase (1.0 mg/ml Sigma-Aldrich) in HBSS (Lonza Ibérica) for 1 h at 37°C, and red blood cells were lysed with an erythrocyte lysis buffer for 5 minutes (Qiagen). The resulting cell suspensions were cultured overnight in DMEM medium supplemented with 10 % foetal bovine serum (Hyclone), and penicillin-streptomycin (Gibco^{™}), at 37 °C and 5% CO₂.

Cells were cultured on round coverslips in 6-well plates and/or 8-well chambered coverglass (Nalge Nunc International) coated with Matrigel. After incubation under the indicated conditions, cells were washed with 1 × PBS and fixed with 4% paraformaldehyde in PBS for 15 minutes, and then permeabilised with 0.1% Triton X-100 in PBS for 10 minutes at room temperature. Permeabilised cells were blocked with 5% bovine serum albumin and 10% normal goat serum in PBS for 15 minutes at room temperature. Thereafter, cultured cells were incubated with primary antibodies. Primary antibodies were incubated over night at 4_{°}C. The following day, slides were washed three times with PBS and incubated 1 hour with secondary antibody. 5-7µm-thick stacks were analysed using a Leica Sp5 for cilia quantification.

### Example 1.6. Western blot and antibodies

Pituitary tissue samples were disaggregated and resuspended in RIPA buffer (Sigma-Aldrich) containing a protease Inhibitor (HaltTM, Thermo-Fisher Scientific). After 30 minutes on ice, samples were sonicated, lysates were centrifuged at 12,700 rpm for 10 minutes and the supernatant was recovered. Western blots were performed using 4-15% Precast Protein Gels, 12-well (Mini-PROTEAN^{®} TGX^{™}), protein homogenates were lysed in Laemmli buffer and the membranes were probed with rabbit polyclonal antisera raised against Arl13B (Proteintech) or β-actin (Santa Cruz Biotechnology). Immunolabelling was detected by enhanced chemiluminescence (SuperSignal West Femto Maximum Sensitivity Substrate, Thermo Scientific) and visualised with a digital luminescent image analyser (Image Quant LAS400 mini).

### Example 1.7. Transcriptome in silico analysis

For transcriptome analysis, dataset from the Gene Expression Omnibus (GEO), accession number GSE63357 was downloaded. From the database, microarray data from 3 sporadic somatotropinomas, 4 sporadic NF-PitNETs and 5 normal glands were analysed. Briefly, a log2 transformation was applied to all samples (excluding fail samples). In case of more than one probe per gene, we collapsed all probe sets by calculating the mean expression. Then, data was grouped and categorised by principal component analysis to identify possible outliers samples. For differential expression between samples, empirical Bayes methods were applied using Limma methodology. FoldChange threshold of 2 and pValue adjusted by False Discovery Rate (FDR) of 0.05 were used for gene selection. Functional enrichment analysis of differential gene expression was performed using clusterProfiler v3.18.1, with a threshold p-value adjusted by FDR of 0.05. We selected all statistically significant categories present in GO, KEGG database and REACTOME

### Example 1.8. Statistical analysis

Quantitative variables are expressed as the median and interquartile range (box and whisker-plots). Mann-Whitney U test was used to compare two independent groups and One-way ANOVA was employed to compare more than two groups and post-hoc multiple comparisons were made with Tukey's test. Spearman's rho analyses were performed to detect correlations between the different markers examined by immunohistochemistry/immunofluorescence. Samples from all groups within an experiment were processed at the same time. The p-values were two sided and statistical significance was considered when *p*<0.05. Data are presented with the specific *p*-values: ^{∗} = *p*<0.05, ^{∗∗} = *p*<0.01, ^{∗∗∗} = *p*<0.005 and ^{∗∗∗∗} = *p*<0.001. All statistical analyses were performed using GraphPad Prism 4 and R v 3.5.1 software.

### Example 2. RESULTS

### Example 2.1. Ciliogenesis is upregulated in pituitary adenomas, mostly in non-functioning pituitary lesions

We examined ciliogenesis by staining for Arl13B, a marker specific for ciliary membranes. PC were characterised by their appearance (cilia length: normal-like and very short length or - dot like) and frequency as described in the Materials and Methods section. Interestingly, whereas in healthy pituitary glands PC were scant, with a very low Arl13b signal in scattered cells (**Figure 1a**), PitNET cells showed robust staining for this ciliary marker (**Figure 1b**). Remarkably, cells in PitNETs showed different patterns of cilia length, with some tumours showing short length PC with a dot-like appearance. Altogether all PitNETs showed a significant increase of cilia index (CI) when compared to control pituitary glands (CI 6.25 ± 3.6 vs 1.26 ± 0.45; p<0.001, **Figure 1c**). Interestingly, when tumours were classified by their clinical functionality, the presence of ciliated cells was significantly higher in NF-PitNETs than in functional tumours (7.99 ± 3.03 vs 2.92 ± 1.80; p<0.001, **Figure 1d****-g**). This was also corroborated by quantification of Arl13b in pituitary protein extracts by western blot (p<0.001) (**Figure 1h** and **i**).Then, we evaluated the frequency of PC in the adenomas classified by the expression of anterior pituitary hormones (somatotropinomas n=24, Prolactinomas n=11, Null cell adenomas n=15, and Gonadotropinomas n=26) (**Figure 1j****-n**). Interestingly, we found a different pattern of PC expression among the different histopathological groups. The group of somatotropinomas and prolactinomas presented an increased CI compared to the control group (3.29 ± 2.00 [p<0.05] and 5.84 ± 4.03 [p<0.01] vs 1.26 ± 0.44, respectively), with an increased frequency of dot-like cilia. Both null cell adenomas and gonadrotropinomas exhibited the highest CI (6.85 ± 4.05 p<0.005, and 9.04 ± 2.44 *p*<0.001 respectively, compared to normal pituitary glands), with a main increase in frequency of normal-like PC. Moreover, the increase in CI in gonadotropinomas was also significant (p<0.001) when compared to somatotropinomas (**Figure 1o**). Concerning the differences of the cilia index observed between functioning and NF-PitNETs, we developed a double immunofluorescence staining for Arl13b and GH, to identify and characterise the phenotype of ciliated cells in both control and PitNET glands. In healthy tissues, the scattered cilia were mainly observed in GH-negative cells (**Figure 2a**). In contrast, in PitNETs cilia were localised at both GH positive and negative cells (**Figure 2b****-c**). In addition, in the GH3 cell line, some GH positive cells had PC (**Figure 2d**).

### Example 2.2. Aggressive tumours have a greater increase in ciliogenesis

We next evaluated the frequency of PC in relation to their clinical behaviour. First, we classified tumours according to their aggressive features including cavernous sinus invasion in both MRI and/or in surgery, the number of surgeries performed and whether the patients were cured/controlled after surgery or had residual disease / recurrence. CI exhibited a significant increase in tumours showing cavernous sinus invasion (4.98 ± 3.39 without invasion vs 7.37 ± 3.44 with invasion, p<0.01, **Figure 3a**) and in those subjected to higher number of transphenoidal/ transnasal surgeries (5.55 ± 3.66 one surgery vs 7.86 ± 2.93 two or more surgeries, p<0.05, **Figure 3b**). CI was also significantly increased in tumours with residual disease in comparison to tumours with no residual disease (6.92 ± 3.62 vs 3.63 ± 2.39 *p*<0.01 **Figure 3c**). This significant increase was only found in NF- PitNETs but not in functional PitNET (4.12 ± 1.39 cured/ controlled NF-PitNET vs 8.58 ± 2.68 NF-PitNETs with residual disease; p<0.005, **Figure 3d**).

### Example 2.3. Expression of cilia-associated genes is increased in non-functioning pituitary adenomas

Our next approach was to identify ciliary molecular alterations involved in PitNETs including somatotropinomas and NF-PitNETs using a transcriptome dataset from Gene Expression Omnibus (GSE63357). First, we evaluated genes showing significant differential expression between the two groups. The results showed a total of 3,317 genes differentially expressed between somatotropinomas and NF-PitNETs, 1,699 were downregulated and 1,618 were upregulated. Then, we carried out a network analysis to identify potential mechanisms involved in the interaction between the different cellular processes in these pituitary tumours. The most represented network was associated with the Wnt signalling pathway (**Figure 4**). In an attempt to elucidate possible differences in ciliary molecular fingerprint we used two transcriptional signatures of cilia-associated genes. The comparison between the list of genes differentially expressed between somatotropinomas and NF-PitNETs showed a total of 97 differentially expressed cilia-related genes (20 downregulated and 77 upregulated). Interestingly, the most upregulated gene in NF-PitNETs was the Dyslexia Candidate Gene DCDC2, whose overexpression has been associated with the increase in ciliary length and activation of Sonic Hedgehog signalling. Likewise, the Arl13b gene was upregulated in NF-PitNETs when compared to GH-secreting tumours (**Figure 4**)**.**

### Example 2.4. Pituitary adenoma cells show increased activation of Hedgehog pathway

Previous reports showed that PitNETs are related to the transcription factor glioma associated oncogene 1 (Glil), which correlates with activation of the Hh pathway. Given the dependence of Hh signalling on the PC, we further analysed by immunohistochemistry and immunofluorescence both in tissue and in cells the expression of Glil and the presence of PC. In healthy tissue, Gli1 was diffusely expressed across the adenohypophyseal cells with occasional sparse cells with high Gli1 expression (**Figure 5a****-b**). In PitNETs, Gli1 expression was increased with a marked staining in the cell membrane (**Figure 5 f, g, k, 1**)**.** Quantification of staining showed that Gl1 expression was significantly increased in PitNETs when compared to controls (p<0.001, **Figure 5p**)**.** Double immunofluorescence staining of PC and Glil showed that cells with PC usually also expressed Glil, which sometimes appeared accumulated at the end of the PC (**Figure 6**)**.** Moreover, there was a significant positive correlation between the cilia index score and Gli1 expression (**Figure 5q**)**.** PC function has also been related to the stem/progenitor cell transcription factor Sox2. Sox2 expression and its correlation with the presence of PC were analysed. Sox2 expression was nearly negligible in healthy tissues, with a very weak expression in some cell nuclei (**Figure 5c** **and d**)**.** However, in PitNETs there was a generalised increase in Sox2 expression throughout the entire tumour (**Figure 5 h-n**)**.** Quantitative analysis confirmed significant increase of Sox2 expression in PitNETs compared to controls (**Figure 5p**; p<0.001). In addition, in PitNET tissues and primary culture cells, PC also expressed high levels of Sox-2 (**Figure 6**)**.** Moreover, there was a significant positive correlation between the cilia index score and Sox 2 expression (p<0.01, **Figure 5q**)**.** Interestingly, we also found a correlation between the expression of GLi1 and Sox2 (**Figure 5q**)**.**

### Example 2.5. Receiver operating characteristics (ROC) curves for the discriminant analysis

By using Youden's index we determined the optimal cut-off value for the CI to differentiate healthy from PitNETs patients and Functioning vs NF-PitNET. The discriminant validity of the CI was assessed by the area under the receiver operating characteristic (ROC) curve (AUC); specificity, sensitivity, positive predictive value, negative predictive value, positive likelihood ratio, and negative likelihood ratio of the CI were also calculated. The optimal cutoff value to determine high risk groups, and the specificity and sensitivity of that point, were assessed by using the Youden index. Odd ratios (OR), were derived by using a logistic regression that included the outcome and the CI.

The best CI cut-off for disease discrimination was 2.37 with a specificity of 97.05% and a sensitivity of 81.42%. The cut-off value to discriminate PA type was 6 with a specificity of 84.78% and a sensitivity of 95.83%. ROC curve analyses for these cut-offs suggested that both were good discriminators between patients with PA and healthy donors, and between functioning and NF-PA being the areas under the curve (AUC) 0.918 and 0.913 with a specificity of 94.85% and 58.46% and a sensitivity of 46.25% and 82.56%, respectively. Interestingly, higher levels of CI is a significant risk factor on the development of PA and, particularly, NF-PA [Odds Ratio (OR) = 4.61, 95% confidence interval (95%CI): 2.52-10.49 and p=0.0004 for Disease and OR = 0.51 (95%CI: 0.39-0.64) and p<0.0001 for Type]. Logistic regression model parameters are summarised in **Figure 8****.** We also assessed the discriminating potency between aggressive parameters (Cavernous sinus invasion, number of surgeries and residual disease) in PA patients using ROC curve analyses and we confirmed that a CI of -6 was a good discriminator of aggressiveness in PAs (AUC>0.67; p<0.05).

## Claims

1. *In vitro* method for the diagnosis and/or prognosis of pituitary neuroendocrine tumours (PitNETs) in a subject, which comprises assessing the presence of primary cilia in cells from a biological sample obtained from the subject, wherein the identification of a frequency of ciliated cells higher than a pre-established frequency threshold value measured in control subjects, is an indication that the subject is suffering from PitNETs and/or that the subject has a poorer prognosis.

2. *In vitro* method for monitoring the aggressiveness of PitNETs which comprises assessing the presence of primary cilia in cells from a biological sample obtained from the subject, wherein the identification of a frequency of ciliated cells higher than a pre-established frequency threshold value measured in control subjects, is an indication that the disease is more aggressive.

3. *In vitro* method for the differential diagnosis of non-functioning PitNET versus functioning PitNET which comprises assessing the presence of primary cilia in cells from a biological sample obtained from the patient, wherein the identification of a frequency of ciliated cells higher than a pre-established frequency threshold value measured in control subjects, is an indication that the subject is probably suffering from a non-functioning PitNETs.

4. *In vitro* method, according to any of the claims 1 or 2, wherein the PitNET is a non-functioning PitNET.

5. *In vitro* method, according to any of the previous claims, wherein the identification ciliated cells is carried out by immunohistochemical or immunofluorescence methods or by electron microscopy.

6. *In vitro* method, according to any of the previous claims, wherein the identification ciliated cells is carried out by using an antibody against ADP ribosylation factor like GTPase 13B.

7. *In vitro* method, according to any of the previous claims, wherein the biological sample is biopsy obtained from the pituitary tumor.

8. *In vitro* use of primary cilia in cells from a biological sample obtained from the patient for the diagnosis and/or prognosis of PitNETs, for monitoring the aggressiveness of PitNETs, or for the differential diagnosis of non-functioning pituitary adenoma versus functioning pituitary adenoma.

9. *In vitro* use of a kit consisting of tools for the identification of primary cilia in cells from a biological sample obtained from the patient, for the diagnosis and/or prognosis of PitNETs, for monitoring the aggressiveness of PitNETs, or for the differential diagnosis of non-functioning pituitary adenoma versus functioning pituitary adenoma.
